# EUROPEAN PATENT SPECIFICATION

(11) **EP 3 037 379 B1**
(45) Date of publication and mention of the grant of the patent: **11.07.2018**
(21) Application number: 15200383.6
(22) Date of filing: 16.12.2015
(51) Int. Cl.: B67C 7/00, A61L 2/18, A61L 2/20, A61L 2/22

(54) **DEVICE AND METHOD FOR REMOVING RESIDUES OF A STERILIZING AGENT**
VORRICHTUNG UND VERFAHREN ZUM ENTFERNEN VON RESTEN EINES STERILISIERUNGSMITTELS
DISPOSITIF ET PROCEDE DESTINÉ À ÉLIMINER DES RESTES D'UN AGENT DE STERILISATION

(30) Priority: 22.12.2014 IT PR20140092
(43) Date of publication of application: 29.06.2016
(73) Proprietor: Gea Procomac S.p.A., 43038 Sala Baganza (PR) (IT)
(72) Inventor: BELLINI, Vittorio, 43017 SAN SECONDO PARMENSE (PR) (IT); COMANI, Andrea, 43038 SALA BAGANZA (PR) (IT)
(74) Representative: Dondi, Silvia

(56) References cited:
- DE-A1- 19 942 507
- DE-A1-102006 036 763
- JP-A- 2003 261 198

## Description

The present invention relates to a device and a method for removing residues of a sterilizing agent from the neck or from a spout of a container for fluid foods.

The reference sector is the bottling of so-called "sensitive" food products, i.e. products that are particularly sensitive to bacteriological contamination and oxidation, such as, for example, isotonic drinks, juices, nectars, soft drinks, teas, milk-based drinks, coffee-based drinks, etc., for which it is fundamental to prevent any microbiological contamination throughout the packaging stages.

Packaging lines using aseptic technology are already known wherein the various operations take place in a controlled-contamination environment, so that the bottled products can be stored for a prolonged period of time and have chemical/physical and organoleptic stability even at room temperature.

In this context, the focus is on the chemical decontamination of the primary packaging, whether it be a bottle (or initial preform), a pouch, a package or a cardboard container. Decontamination of the primary packaging takes place before the latter is filled.

One of chemical decontamination techniques known to date provides for the inside walls of the container to be treated by means of an aqueous solution containing a sterilizing agent (e.g. hydrogen peroxide or peracetic acid).

As is well known, container sterilization performance is expressed by the number of decimal reductions that the sterilization treatment is capable of achieving against a reference microorganism. For example, for aseptic lines that package low-acid products, six decimal reductions are generally required, whereas in lines that handle high-acid products, four decimal reductions are sufficient.

On the other hand, because of regulatory requirements, the residue of sterilizing agent present inside the beverages must remain below a preestablished threshold (typically < 0.5 ppm). For this reason, after the treatment with sterilizing solution there is a step of rinsing the container with air or water, followed by one of filling the container with the beverage. The rinsing takes place by means of nozzles that dispense the air or water onto the inside surface of the container. In particular, the nozzles of a stretch-blow moulding machine can be used directly.

For example, the rinsing of containers with water after treatment with hydrogen peroxide is described in document US7900422.

It is clear that low-acid beverages, as they require higher concentrations of the sterilizing agent, pose greater problems for the removal of the residual sterilizing agent.

In fact, during sterilization with hydrogen peroxide, an effect of mass diffusion of the hydrogen peroxide inside the mould for the PET of the preform or of the container may occur.

As is well known, the mass diffusivity or permeability of a fluid in a solid depends above all on the nature of these materials. In particular, PET has a molecular structure that can be penetrated by hydrogen peroxide.

The mass diffusivity further depends on the pressure of the fluid and the temperature of the solid. The latter undoubtedly has relevance in applications providing for sterilization of the preform. In fact, the preform is heated to about 100°C in order to be subsequently moulded, for example by stretch-blowing.

Sterilization with hydrogen peroxide vapours makes it possible to reduce the residues of sterilizing agent compared to sterilization performed with peroxide vapours in the condensed phase.

However, even if present in reduced amounts, such residues can be deposited on the outer surface of the neck of the container and thus be perceived by the user at the time of consuming the beverage, through direct contact between lips and neck. The residues of the sterilizing agent on the outer surface of the neck can reach concentrations above 100 ppm.

A known solution envisages removing any filling product residues from the outer area of the neck after the filling step. This removal is carried out by washing the neck with sterile water, which possesses a high diffusion capacity in PET. Therefore, besides removing filling product residues from the neck, the washing water is also able to extract the peroxide trapped in the PET. After washing with sterile water, the step of capping the container takes place. The drops of water remaining on the neck following washing give rise to layers or films of water due to the rubbing exerted by the cap on the outer thread of the neck of the container.

The spontaneous evaporation of the aqueous films nonetheless leaves peroxide residues on the outer neck (concentrations of around 20-25 ppm), which, in contact with a consumer's lips, generate a slight sensation of irritation.

DE19942507 discloses a device in accordance with the preamble of claim 7, and a method for closing containers that includes a cleaning step after fitting on a sealing insert. The goal of the sealing insert is to prevent the cleaning liquid to enter the container. Then, a drying step takes place and a screw cap is fitted on. According to this solution, cleaning and drying affect only the lateral surface of the neck, thus resulting in a not complete treatment of the outer surface ot the neck. In this context, the technical task at the basis of the present invention is to propose a device and a method for removing residues of a sterilizing agent from the neck or from a spout of a container for fluid foods which overcomes the aforementioned drawbacks of the prior art.

In particular, the object of the present invention is to propose a device and a method for removing residues of a sterilizing agent from the neck or from a spout of a container for fluid foods in an effective manner, so that the consumer no longer perceives any residues on the outer surface of the neck or spout at the time of consuming the product.

The stated technical task and specified objects are substantially achieved by a method for removing residues of a sterilizing agent from the neck or from a spout of a container for fluid foods according to claim 1. Preferably, the step of washing the outer surface of the neck or spout of the container takes place before, after or during a step of filling the container.

Preferably, the step of drying the outer surface of the neck or spout of the container takes place after filling and before capping the container. Preferably, the sterile gas dispensed over the outer surface of the neck or spout of the container during the drying step has a temperature of between 15°C and 120°C.

Preferably, the sterile gas dispensed over the outer surface of the neck or spout of the container is sterile compressed air.

Preferably, the neck or spout of the container is made of PET and the sterile washing fluid is in a liquid phase so as to spread easily in the structure of the PET. During the step of drying the outer surface of the neck or spout of the container, the flow of sterile gas is directed so as not to enter the mouth of the open container.

The stated technical task and specified objects are substantially achieved by a device for removing residues of a sterilizing agent from the neck or spout of a container for fluid foods according to claim 7. Preferably, the nozzles of the second series are not located above the mouth of the open container.

Preferably, the nozzles of the second series are arranged laterally relative to the neck or spout of the container.

Further features and advantages of the present invention will be more apparent from the approximate, and hence non-limiting description of a preferred, but not exclusive, embodiment of a device and a method for removing residues of sterilizing agent from the neck or from a spout of a container for fluid foods, as illustrated in the appended drawings, in which:
- figure 1 illustrates a line for the production and packaging of containers comprising a device for removing residues of a sterilizing agent from the neck or from a spout of a container for fluid foods, according to the present invention, in a plan view (part of the outer casing of the unit has been removed for the sake of clarity);

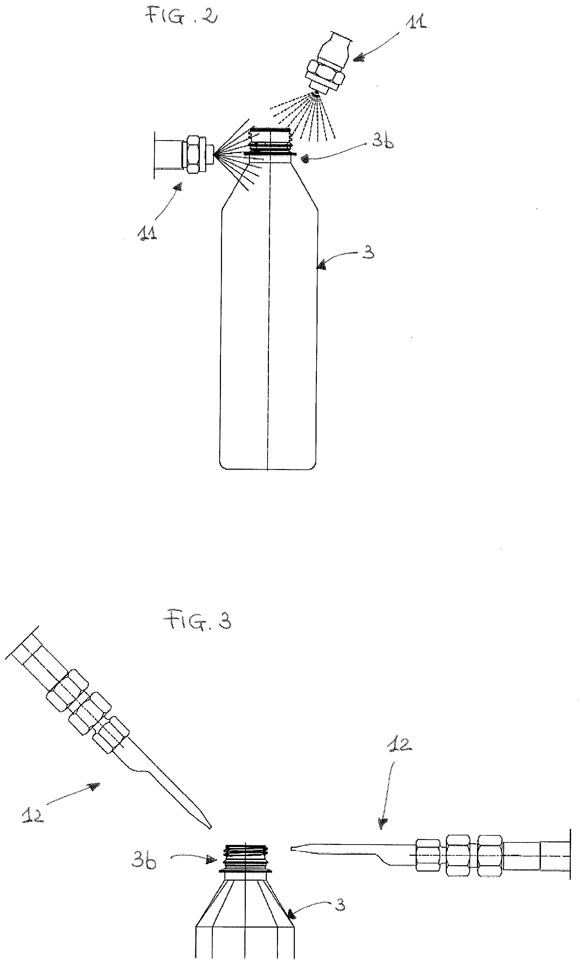

- figure 2 schematically illustrates a container and a part (first series of nozzles) of the device for removing residues of a sterilizing agent from a neck or spout, according to the present invention;
- figure 3 schematically illustrates a container and a part (second series of nozzles) of the device for removing residues of a sterilizing agent, according to the present invention.

With reference to the figures, the number 1 denotes a line for the production and packaging of containers 3.

In the embodiment described and illustrated here, the containers 3 are obtained from preforms made of thermoplastic material. Preferably, the preforms are made of PET.

Preferably, each preform has a tubular body and a neck which do not undergo processing. In the description below, the identification number 3b will be used to indicate the neck of the moulded container 3, which thus also corresponds to the neck of the preform.

In an alternative embodiment (not illustrated), the containers 3 are what are known in technical jargon as pouches. Each container 3 is provided with what in technical jargon is known as a spout.

In the embodiment described and illustrated here, the production and packaging line 1 comprises:
- a unit 5 for heating the preforms 2;
- a unit 6 for chemically sterilizing the preforms 2;
- a unit 7 for forming the containers 3 from the sterilised preforms 2;
- a unit 8 for filling the formed containers 3;
- a unit 9 for closure or capping of the filled containers 3.

On the outlet side of the heating unit 5, the preforms have a temperature of around 100°C. In the chemical sterilization unit 6, the preforms are subjected to a flow of hydrogen peroxide vapours.

Preferably, the forming unit 7 is an aseptic blow-moulding unit.

In the preferred embodiment, the filling unit 8 consists in an aseptic filler of low-acid products.

The production and packaging line 1 also comprises a device 10 for removing residues of a sterilizing agent from the neck 3b of the containers 3. The device 10 for removing the residues of a sterilizing agent comprises:
- a first series of nozzles 11 for dispensing a sterile fluid;
- a second series of nozzles 12 for dispensing a sterile gas.

In particular, the first series of nozzles 11 is arranged downstream of the sterilization unit 6 relative to the feed path of preforms and containers along the production and packaging line 1. For example, the first series of nozzles 11 is arranged upstream or downstream of the filling unit 8. In a variant embodiment, some nozzles 11 of the first series are located upstream and others are located downstream of the filling unit 8. The nozzles 11 of the first series are arranged in such a way as to dispense the sterile fluid on the outer surface of the neck 3b of the containers 3.

Preferably, the sterile fluid dispensed by the nozzles 11 of the first series is in a liquid phase so as to spread easily in the structure of the PET of the neck 2b.

Preferably, the nozzles 11 of the first series are mounted on a transfer star wheel 13.

The second series of nozzles 12 is arranged downstream of the filling unit 8 and upstream of the closure unit 9 relative to the feed path of preforms and containers along the production and packaging line 1. The nozzles 12 of the second series are arranged in such a way as to dispense a sterile gas over the outer surface of the neck 3b of the containers 3 in order to dry them. Furthermore, the nozzles 12 of the second series are arranged in such a way as not to affect the inside of the container 3, in order to prevent the product contained therein from leaking out.

The nozzles 12 of the second series are arranged in such a way that the jet of sterile gas dispensed by them does not enter the mouth of the open container 3.

In particular, the nozzles 12 of the second series are not above the mouth of the open container 3.

For example, the nozzles 12 of the second series are arranged laterally to the neck 3b of the container 3.

Preferably, the sterile gas dispensed by the nozzles 12 of the second series has a temperature comprised between 15°C and 120°C.

For example, the sterile gas is sterile compressed air.

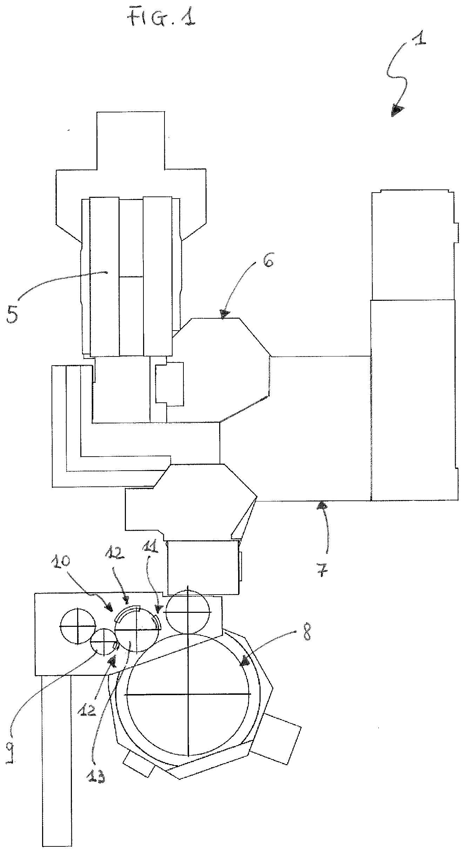

In the embodiment in figure 1, the nozzles 12 of the second series are mounted on the same transfer star wheel 13 as the nozzles 11 of the first series.

Alternatively, the nozzles 12 of the second series are mounted on a further transfer star wheel interposed between the filling unit 8 and closure unit 9. In a variant embodiment, some nozzles 12 of the second series are arranged on the closure unit 9.

As already said above, in a further embodiment the containers 3 are pouches provided with spouts.

In such a case, the production and packaging line 1 comprises:
- a unit for sterilizing the spouts;
- a unit for forming the pouches;
- a unit for filling the pouches;
- a unit for closing or capping of the pouches.

There is also a device 10 for removing residues of sterilizing agent from the spouts provided on the pouches. The device 10 for removing residues of sterilizing agent comprises:
- a first series of nozzles 11 for dispensing a sterile fluid;
- a second series of nozzles 12 for dispensing a sterile gas.

In particular, the first series of nozzles 11 is arranged downstream of the spout sterilization unit relative to the feed path of the pouches along the production and packaging line 1. For example, the first series of nozzles 11 is arranged upstream or downstream of the filling unit. In a variant embodiment, some nozzles 11 of the first series are located upstream and others are located downstream of the filling unit. The nozzles 11 of the first series are arranged in such a way as to dispense the sterile fluid over the outer surface of the spouts. Preferably, the sterile fluid dispensed by the nozzles 11 of the first series is in a liquid phase so as to spread easily in the structure of the PET of the spouts.

The second series of nozzles 12 is located downstream of the filling unit and upstream of the closing unit relative to the feed path of the pouches along the production and packaging line 1. The nozzles 12 of the second series are arranged in such a way as to dispense the sterile gas over the outer surface of the spouts in order to dry them.

For example, the nozzles 12 of the second series are arranged laterally to the spouts.

Preferably, the sterile gas dispensed by the nozzles 12 of the second series has a temperature comprised between 15°C and 120°C.

For example, the sterile gas is sterile compressed air.

The method for removing residues of sterilizing agent from the neck of a container for fluid foods is described below. In this case reference is made to a container 3 obtained from a preform made of PET.

Let us consider a container 3 which has already been sterilized and formed from the preform. Because of the sterilization, residues of a sterilizing agent, for example hydrogen peroxide, are present in the neck 3b of the container 3.

The outer surface of the neck 3b then undergoes a step of washing with a sterile fluid, for example sterile water, coming from the first series of nozzles 11.

The use of a sterile fluid in a liquid phase in fact facilitates penetration into the structure of the PET, enabling extraction of the sterilizing agent trapped in the neck 3b.

The step of washing with a sterile fluid can take place before, after or during a step of filling the container 3. The outer surface of the neck 3b of the container 3 undergoes a step of drying by means of the delivery of a sterile gas, for example sterile compressed air, by the second series of nozzles 12. The drying step takes place before the step of closing or capping the container 3.

During the drying step, the flow of sterile gas is directed in such a way as not to enter the mouth of the open container 3.

From the description given, the features of the device and method for removing residues of a sterilizing agent from the neck or from a spout of a container according to the present invention appear clear, as do the advantages thereof.

In particular, introducing a step of drying with sterile gas after the step of washing the neck (or spout) enables the residues of a sterilizing agent which tend to accumulate on the outer surface of the neck (or spout) to be efficiently removed.

These residues remain at concentrations of around 5 ppm, compatible with direct consumption from the neck of the container.

## Claims

1. Method for removing residues of a sterilizing agent from the neck (3b) or from a spout of a container (3) for fluid foods, comprising the step of:
washing the outer surface of the neck (3b) or spout of said container (3) by means of a sterile fluid coming from a first series of nozzles (11);
after washing, drying the outer surface of the neck (3b) or spout (4) of said container (3) by dispensing a flow of sterile gas coming from a second series of nozzles (12) over said outer surface, and said step of drying takes place before a step of closing or capping the container (3), whereby during the step of drying the outer surface of the neck (3b) or spout (4) of said container (3) the flow of sterile gas is directed in such a way as not to enter the mouth of the open container (3), in order to prevent the fluid food contained therein from leaking out.

2. Method according to claim 1, wherein said step of washing the outer surface of the neck (3b) or spout of the container (3) takes place before, after or during a step of filling the container (3).

3. Method according to claim 1 or 2, wherein said step of drying the outer surface of the neck (3b) or spout of the container (3) takes place after filling and before capping said container (3).

4. Method according to any one of the preceding claims, wherein the sterile gas dispensed over the outer surface of the neck (3b) or spout of the container (3) during the drying step has a temperature of between 15°C and 120°C.

5. Method according to any one of the preceding claims, wherein the sterile gas dispensed over the outer surface of the neck (3b) or spout of the container (3) is sterile compressed air.

6. Method according to any one of the preceding claims, wherein said neck (3b) or spout of the container (3) is made of PET, said sterile washing fluid being in a liquid phase so as to spread easily in the structure of the PET.

7. Device (10) for removing residues of a sterilizing agent from the neck (3b) or from a spout of a container (3) for fluid foods using a method according to claim 1 to 6, **characterised in that** the device comprises:
a first series of nozzles (11) arranged in such a way as to dispense a sterile fluid over the outer surface of the neck (3b) or spout of said container (3);
a second series of nozzles (12) arranged in such a way as to dispense a sterile gas over the outer surface of the neck (3b) or spout of said container (3) containing fluid food and not provided with a closure or cap, and **in that** the nozzles (12) of the second series are arranged in such a way as not to affect the inside of the open container (3), in order to prevent the fluid food contained therein from leaking out, wherein the nozzles (12) of the second series are arranged in such a way that the jet of sterile gas dispensed by them does not enter the mouth of the open container (3).

8. Device (10) according to claim 7, wherein the nozzles (12) of the second series are not located above the mouth of the open container (3).

9. Device (10) according to claim 7 or 8, wherein the nozzles (12) of the second series are arranged laterally relative to the neck (3b) or spout of the container (3).

## Patentansprüche

1. Verfahren zum Entfernen von Resten eines Sterilisierungsmittels vom Hals (3b) oder von einer Ausgusstülle eines Behälters (3) für flüssige Nahrung, umfassend den Schritt:
Waschen der Außenfläche des Halses (3b) oder der Ausgusstülle des Behälters (3) mittels einer sterilen Flüssigkeit, die aus einer ersten Reihe von Düsen (11) stammt;
Trocknen nach dem Waschen der Außenfläche des Halses (3b) oder der Ausgusstülle (4) des Behälters (3), indem ein Strom von Sterilgas aus einer zweiten Reihe von Düsen (12) über die Außenfläche abgegeben wird, und der Schritt des Trocknens vor einem Schritt des Verschließens oder des Versehens des Behälters (3) mit einer Kappe erfolgt, wobei während des Schrittes des Trocknens der Außenfläche des Halses (3b) oder der Ausgusstülle (4) des Behälters (3) der Strom des Sterilgases so geleitet wird, dass er in den Mund des offenen Behälters (3) nicht gelangt, um zu verhindern, dass die darin enthaltene flüssige Nahrung austritt.

2. Verfahren nach Anspruch 1, wobei der Schritt des Waschens der Außenfläche des Halses (3b) oder der Ausgusstülle des Behälters (3) vor, nach oder während eines Füllschritts des Behälters (3) erfolgt.

3. Verfahren nach Anspruch 1 oder 2, wobei der Schritt des Trocknens der Außenfläche des Halses (3b) oder der Ausgusstülle des Behälters (3) nach dem Befüllen und vor dem Versehen des Behälters (3) mit der Kappe erfolgt.

4. Verfahren nach einem der vorhergehenden Ansprüche, wobei das während des Schritts des Trocknens über die Außenfläche des Halses (3b) oder der Ausgusstülle des Behälters (3) abgegebene Sterilgas eine Temperatur zwischen 15 °C und 120 °C aufweist.

5. Verfahren nach einem der vorhergehenden Ansprüche, wobei das über die Außenfläche des Halses (3b) oder Ausgusstülle des Behälters (3) abgegebene Sterilgas sterile Druckluft ist.

6. Verfahren nach einem der vorhergehenden Ansprüche, wobei der Hals (3b) oder Ausgusstülle des Behälters (3) aus PET hergestellt ist, wobei die sterile Waschflüssigkeit in einer flüssigen Phase ist, um sich leicht in der PET-Struktur zu verteilen.

7. Vorrichtung (10) zum Entfernen von Resten eines Sterilisierungsmittels vom Hals (3b) oder von einer Ausgusstülle eines Behälters (3) für flüssige Nahrung unter Verwendung von einem Verfahren nach Anspruch 1 bis 6, **dadurch gekennzeichnet, dass** die Vorrichtung umfasst:
eine erste Reihe von Düsen (11), die so angeordnet sind, dass sie eine sterile Flüssigkeit über die Außenfläche des Halses (3b) oder der Ausgusstülle des Behälters (3) abgeben;
eine zweite Reihe von Düsen (12), die so angeordnet sind, dass sie ein Sterilgas über die Außenfläche des Halses (3b) oder der Ausgusstülle des Behälters (3) abgeben, der flüssige Nahrung enthält und nicht mit einem Verschluss oder einer Kappe versehen ist, und dass die Düsen (12) der zweiten Reihe so angeordnet sind, dass sie das Innere des offenen Behälters (3) nicht beeinflussen, so dass die darin enthaltene flüssige Nahrung nicht austritt, wobei die Düsen (12) der zweiten Reihe so angeordnet sind, dass der von ihnen abgegebene Sterilgasstrahl nicht in den Mund des offenen Behälters (3) gelangt.

8. Vorrichtung (10) nach Anspruch 7, wobei die Düsen (12) der zweiten Reihe nicht oberhalb des Mundes des offenen Behälters (3) angeordnet sind.

9. Vorrichtung (10) nach Anspruch 7 oder 8, wobei die Düsen der zweiten Reihe seitlich zum Hals (3b) oder Ausgusstülle des Behälters angeordnet sind.

## Revendications

1. Procédé destiné à éliminer des restes d'un agent de stérilisation sur le col (3b) ou sur un bec d'un récipient (3) pour fluides alimentaires, comprenant l'étape :
laver la surface externe du col (3b) ou bec dudit récipient (3) avec un fluide stérile provenant d'une première série de buses (11);
après le lavage, sécher la surface externe du col (3b) ou bec (4) dudit récipient (3) en distribuant un flux de gaz stérile provenant d'une seconde série de buses (12) sur ladite surface externe, et ladite étape de séchage se produisant avant une étape de fermeture ou capsulage du récipient (3), dans lequel, pendant l'étape de séchage de la surface externe du col (3b) ou bec (4) dudit récipient (3), le flux de gaz stérile est orienté de sorte à ne pas entrer par le goulot du récipient ouvert (3), pour empêcher que ne s'échappe le fluide alimentaire contenu à l'intérieur.

2. Procédé selon la revendication 1, dans lequel ladite étape de lavage de la surface externe du col (3b) ou bec du récipient (3) se produit avant, après ou pendant une étape de remplissage du récipient (3).

3. Procédé selon la revendication 1 ou 2, dans lequel ladite étape de séchage de la surface externe du col (3b) ou bec du récipient (3) se produit après le remplissage et avant le capsulage dudit récipient (3).

4. Procédé selon l'une quelconque des revendications précédentes, dans lequel le gaz stérile distribué sur la surface externe du col (3b) ou bec du récipient (3) pendant l'étape de séchage a une température entre 15 °C et 120 °C.

5. Procédé selon l'une quelconque des revendications précédentes, dans lequel le gaz stérile distribué sur la surface externe du col (3b) ou bec du récipient (3) est de l'air comprimé stérile.

6. Procédé selon l'une quelconque des revendications précédentes, dans lequel ledit col (3b) ou bec du récipient (3) est fabriqué en PET, ledit fluide stérile de lavage étant dans une phase liquide pour se répandre facilement dans la structure du PET.

7. Dispositif (10) destiné à éliminer des restes d'un agent de stérilisation sur le col (3b) ou sur un bec d'un récipient (3) pour fluides alimentaires, appliquant un procédé selon les revendications 1 à 6, **caractérisé en ce qu'**il comprend :
une première série de buses (11) disposées de sorte à distribuer un fluide stérile sur la surface externe du col (3b) ou bec dudit récipient (3) ;
une seconde série de buses (12) disposées de sorte à distribuer un gaz stérile sur la surface externe du col (3b) ou bec dudit récipient (3) contenant un fluide alimentaire et non muni d'une fermeture ou capsule, et **en ce que** les buses (12) de la seconde série sont disposées de sorte à ne pas toucher l'intérieur du récipient ouvert (3), pour empêcher que ne s'échappe le fluide alimentaire contenu à l'intérieur, dans lequel les buses (12) de la seconde série sont disposées de sorte que le jet de gaz stérile distribué par elles n'entre pas par le goulot du récipient ouvert (3).

8. Dispositif (10) selon la revendication 7, dans lequel les buses (12) de la seconde série ne sont pas situées au-dessus du goulot du récipient ouvert (3).

9. Dispositif (10) selon les revendications 7 ou 8, dans lequel les buses (12) de la seconde série sont disposées latéralement par rapport au col (3b) ou bec du récipient (3).
